Europäisches Patentamt

European Patent Office

Office européen des brevets

(11) Publication number: **0 154 102**
**A2**

(12) # EUROPEAN PATENT APPLICATION

(21) Application number: **84830292.3**

(22) Date of filing: **29.10.84**

(51) Int. Cl.⁴: **A 61 F 5/02**
**A 61 N 1/22**

(30) Priority: **31.10.83 IT 954883**

(43) Date of publication of application:
**11.09.85 Bulletin 85/37**

(84) Designated Contracting States:
**AT DE FR GB NL SE**

(71) Applicant: **Talluri, Antonio**
**Sezzate, 20**
**I-50027 Strada in Chanti (Florence)(IT)**

(72) Inventor: **Talluri, Antonio**
**Sezzate, 20**
**I-50027 Strada in Chanti (Florence)(IT)**

(54) **Autocorrective side shift exercise incentivated by external apparatus, for the treatment of adolescent idiopathic scoliosis.**

(57) To correct the spine deformities, in particular to correct the scoliosis by lateral translation, it is foreseen the generation of stimuli apt to incentivate in the subject the correction of the spine by lateral shift obtained with ones muscles, and to induce such stimuli on the patient only if and when the same is not in a suitable position of the back to have a correction of the deformity, and also in such a way to interrupt the stimuli if and when the patient assumes the required position of the back for a correct treatment. An apparatus for this purposes utilized the trunk deformity.

FIG. 2

## DESCRIPTION

In Orthopaedics is known, amongst the various spine deformities, one constituded by a lateral deviation of the spine, named idiopathic scoliosis. Other deformities are known such as lordosis, that is an excess of curvature (with posterior concavity) of the lower part of the spine.

The invention is referred to a method to correct mainly the scoliosis, and also secondarly the lordosis.

Scientifically has been proven that a correction of the scoliotic curve is obtained soliciting the trunk to a side shift in the opposite direction of the deformity of the scoliotic malformation; the patient must try to move the trunk with its own musculature to obtain with the side shift a correct posture, which if it is repeated numerous times, and mantained, can arrest the progression of such disease (progressive idiopathic scoliosis).

This is not easy because the subject trend to neglect the exercise and to reduce it in duration, because of the fatigue and stress, with the consequence that the correction by side shift results then practically little effective. A scrupolous compliance to the method will bring the patient to support more and more easly the stress of the side shift, increasing this way the duration of successive treatments, interrupted by some rest timing, thus increasing the efficacy of the correction by side shift.

The method of this invention propose firstly to allow to reach an effective scoliosis treatment by a lateral translation (side shift).

The invention will cause also, secondarly to impose and exercise to correct the lordosis, in concomitance with the scoliosis corrective one (pelvis tilt).

Substantially accordingly to the invention the method for treating scoliosis with a side shift foreseens to generate stimuli capable to incentivate the subject to the autocorrection of the spine by side shift throught the muscles; the stimuli are induced on the patient only if and when the same is not already in the required position for the correction and also in such a way to cut off the stimulus if and when the patient assumes the desired position for the treatment.

Therefore the patient is stimulated to assume the required position for the treatment, in such a way to interrupt the stimulus.

The generated stimulus can be adjusted to a suitable amplitude, to graduate the noxiceptivnes of the stimulus.

The stimuli, mainly of electric nature and mainly locally induced, are advantageously generated with preset frequence and/or duration that may be adjusted in relation to the supportability of the muscular exercise required for the treatment.

The stimuli can be applied posteriorly, but also as an alternative anteriorly in order to induce also a pelvic tilt that obtain the correction of the lordosis.

The invention relates also an apparatus to abtuate the above-mentioned method. Such apparatus includes a box that is attached locally over the patient's trunk, proximal to the area that must be subject to the deformation for the side shift treatment (the lumbosacral hinge) and from such box it leans out an apposite appendix, relatively long, that is affixed to the trunk on the area that with the side shift can be moved in such a way to obtain a signal in a system, mainly electric or electronic in absence and respectively in presence of a side corrective shift.

The apparatus can also include electrodes applied to the skin to provoke the stimulus in function of a predisposition that includes a preset frequency and duration of the stimuli, in function of the feedback signal originated by said appendix.

Practically tha apparatus include a timer to determine the frequency of the generation of the stimuli and an adjustement for the duration of the stimuli, as well as a system to adjust the amplitude of the stimuli.

The aforementioned appendix can be formed by a rod, articulated on the case and affixed to the torso, so to obtain a movement in presence of the correction imparted to the trunk for the corrective treatment. By adjusting the positioning of said appendix, it is possible to determine the cut off the stimulus only in presence of a side shift of the upper trunk, at a certain limit that can be varied upon judgement of the expert.

The apparatus or the relevant electrodes can be affixed on the back, particularly on the pelvis as far as the case is concerned.

Nevertheless the apparatus can also be applied anteriorly or at least could be applied anteriorly the electrodes that induce the stimulus, when the pelvic tilt is requested to correct the lordosis; in fact in such instance the patient on top of being stimulated to the lateral translaction to correct the scolios, is stimulated to a pelvic tilt, in the attempt to run away from the anterior stimulus, achieving this way a correction of the lordosis.

The invention will be better understood following the description and the enclosed drawing, which shows a non limitative practical example of the invention.

In the drawing figure N. 1 and 2 show the pattern of the spine in case of scoliosis and respectively in the conditions
of side shift for the correction;
Fig. 3 shows from the back, a way to apply the apparatus;
Fig. 4 shows a vertical section by IV-IV of fig. 3;
Fig. 5 shows an horizontal section at V-V of fig. 4; and
Fig. 6 shows a prospective of the sole apparatus.

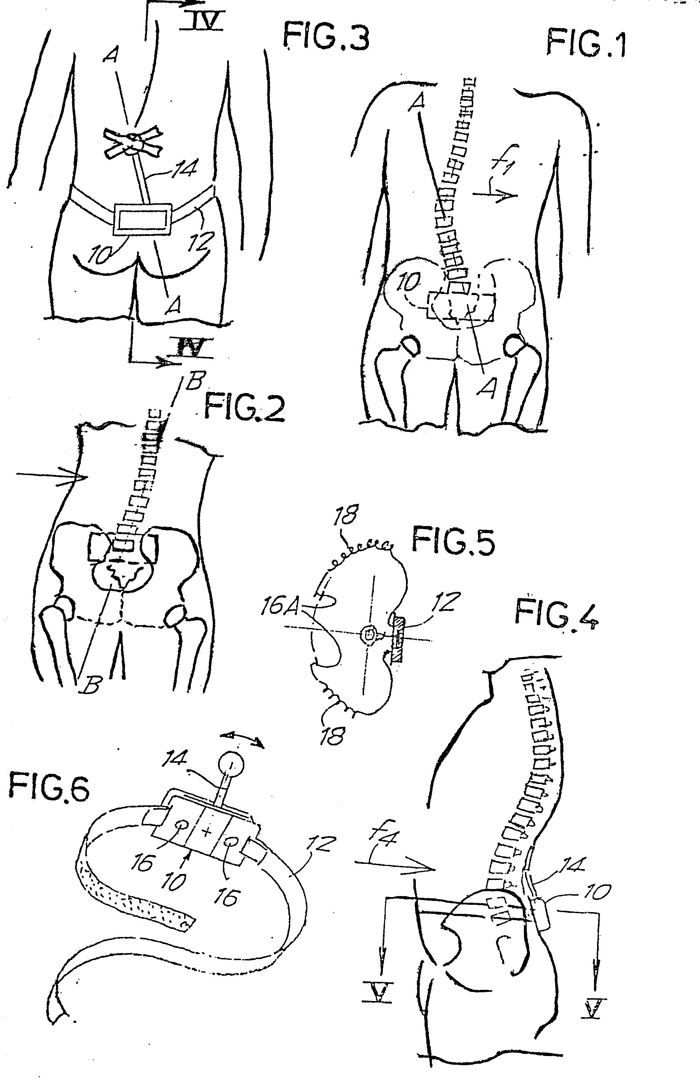

For the correction and the therapy of the scoliosis it is
suggested a lateral translation of the trunk in accordance
to the arrow f1 of figure 1 to bring the spine from the pattern of fig. 1 to the pattern of fig. 2.

This can be obtained other than with a brace, voluntarly
by the patient with a muscular exercise that must be repeated
and prolonged to the maximum possible time.
This is not easy mainly in case of young patients, for which
the therapy of side shift result to be particularly effective.
To obtain a stimulation to the side shift, and therefore
the efficacy of the therapy, it is foreseen the subjected
apparatus.
This may include a container 10 applicable with belting systems
12, with adhesive tapes or other to the back in correspondence
of the pelvic structure and the sacral tract, in such a way
that it cannot participate to the movements of the spine.
From the container 10 it extends an appendix 14 that can
angularly move inside the container and subject to be affixed
to the back above and distal from the area of application
of the container 10, in such a way that the rod 14 can make
an angular excursion for example from the line A-A of the
figures 1 and 3 to the line B-B of figure 2, when the trunk
is shifted laterally by the patient to achieve the correction.
To the rod 14 is associated a sensing system, for example
a magnetic proximity switch (reed) or of other nature (even
optical eventually) in order to detect the reaching of the
position of Fig. 2 respectively and the permanence in the
patterns of figures 1 or 3.
An internal circuit is provided to generate priodical stimuli,
adjustable per frequency and duration, whose emission is
governed and controlled from the sensor combined to the rod
14.

With such the stimulus is delivered only at the frequency and for the duration prescribed and furthermore only if the patient has not performed the side shift of its own trunk from the pattern of fig. 1 to the one of fig. 2.

The position of the rod 14 can be adjusted to regulate the slope of the A-A axis in the resting pattern, and also the lenght of the rod 14 can be modified, and respectively can be modified the geometrical or the electrical conditions to establish at demand the sensitivity of the sensing mean represented by the rod 14 that performs the excursion.

For the application of the stimulus to the patient can be for example foreseen a pair of electrodes 16 directly carried by the box 10, but is not excluded that the electrodes can be carried by free leads as in 18, to apply the electrodes 16A in different positions from the ones of the box.

In this way for example the electrodes could be applied for the anterior stimulus, in order to induce the patient to instinctively move as of the arrow f4 of Fig. 4 with a pelvic tilt to correct the lordosis, or the excess of the lower curve with back concavity, that is frequently associated to the scoliosis.

It can also be foreseen, in this lordosis evenience, to apply the box 10 with the rod 14 in the front of the body with analogous functions.

The stimuli of electric nature, other that be adjustable as said per frequency and duration can also be adjusted in amplitude (intensity).

It is possible to provoke with wanted frequencies and durations suitable stimuli to induce the patient to assume the lateral translated position of fig. 2 in which the apparatus thanks to the movement of the rod 14 cuts of the stimulus, which is restored with the return to the pattern of fig. 1 but only during the emission cycles of the stimuli (duty cycle).

It is understood that the drawing shows only an example that is given only as a practical demonstration of the invention, being such invention variable in forms and dispositions without in any case outgoing from the concept that informs about the invention itself.

For example the stimuli can be in lieu or other than electrical nature, of other kind (sound, light or other) and can be applied to any other part of the body or anyhow supplied.

-6-

CLAIMS

1) - Method for the correction of spine deformities, in particular for the correction of scoliosis by means of side shift, characterized by the fact to provide the generation of stimuli apt to incentivate  in the subject the correction of the spine by side shift obtained with ones musculature, and the induction of such stimuli on the patient only if and when the patient has not already assumed the position of the trunk that is suitable for the correction, and also in a way to cease the stimulus if and when the patient assumes the desired position of the trunk for the treatment.

2) - Method as per above claim, characterized by the fact that the stimuli generated is adjustable to and adequate intensity.

3) - Method as per claims 1 or 2 characterized by the fact that the stimuli-mainly of electric nature- are provoked -at preset and adjustable frequencies and/or duration, in relation to the supportability of the muscular exercise required for the treatment.

4) - Method as per above claims, characterized by the fact that the stimuli are applied posteriorly.

5) - Method as per Claims 1 to 3, characterized by the fact that the stimuli are applied anteriorly on the ventral region to solicit also a pelvic tilt, with which is obtained a correction of the lordosis.

6) - Method as described and illustrated.

7) - An apparatus to actuate the method as per above claims, characterized by the fact to comprehend: a container applicable locally on the patient's trunk proximally to the zone that must be subject to the deformation for the treatment of side shift; an appendix relatively long leaning out from the container, that is affixed on the trunk over the zone that with the side shift is moving in such a way to obtain a signal with a suitable system, mainly electric or electronic, in absence respectively in presence of a lateral translation of correction; and electrodes applied to the skin to provoke the stimulus in function of a presetting that comports a frequency and a duration of the stimuli set in advance and a consent to the emission of the same in function of the signal of sensing actuated by means of said appendix.

8) - Apparatus as per claim 7, characterized by the fact that it comprehends means to adjust the frequency ot stimuli generation and/or the duration of the stimuli, as well as an intensity regulating mean.

9) - Apparatus as per claim 7 or 8, characterized by the fact that the appendix is formed by a rod (antenna) articulated to the container and applicable to the torso, in such a way to obtain a movement in presence of the correction imposed to the torso for the corrective treatment.

10) - Apparatus as per claims 7 and succesives, characterized by the fact that it comprehends means to vary the position of application of said appendix, to cause the cut-off of the stimuli only in presence of a side shift of the upper trunk at a determined limit, that can be varied at expert's will.

11) - Apparatus as per claim 7 and successives, characterized by the fact that the electrodes are carried by the container.

12) - Apparatus as per claims 7 to 10 characterized by the fact that the electrodes are carried by flexible leads, for a remote application.

13) - Apparatus as described and illustrated.

FIG.3

FIG.1

FIG.2

FIG.5

FIG.4

FIG.6